# DEMANDE DE BREVET EUROPEEN

(11) **EP 0 780 387 A1**
(43) Date de publication de la demande: **25.06.1997**
(21) Numéro de dépôt: 96402660.3
(22) Date de dépôt: 09.12.1996
(51) Int. Cl.: C07D 333/20, A61K 31/38

(54) **Nouveaux composés du thiophène, leur procédé de préparation et les compositions pharmaceutiques qui les contiennent**

(30) Priorité: 21.12.1995 FR 9515224
(71) Demandeur: ADIR ET COMPAGNIE, 92415 Courbevoie Cédex (FR)
(72) Inventeur: Wierzbicki, Michel, 78620 L'Etang la Ville (FR); Sauveur, Frédéric, 95100 Argenteuil (FR); Boussard, Marie-Françoise, 78124 Mareil Sur Mauldre (FR); Bonnet, Jacqueline, 75013 Paris (FR); Sabatini, Massimo, 92380 Garges (FR)
(74) Mandataire: Reverbori, Marcelle

(57) **Abrégé**

Les composés du thiophène de formule : 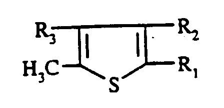 dans laquelle
* un des R₁, R₂ et R₃ représente le radical de formule 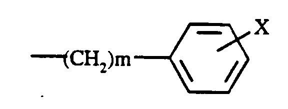 dans laquelle :
   m représente un nombre entier de 2 à 6 inclus et
   X représente un atome d'hydrogène, un radical alkyle ou alkoxy ayant chacun de 1 à 5 atomes de carbone en chaîne droite ou ramifiée, ou un radical dialkylamino dans lequel chaque groupe alkyle renferme de 1 à 5 atomes de carbone,
   un des R₁, R₂ et R₃ représente le radical de formule 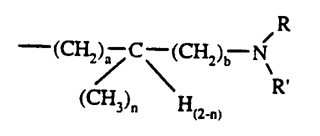
dans laquelle :
- n représente 0, 1 ou 2 ;
- a représente 2 ou 3 et
- b représente 1 ou 2 de telle sorte que a+b=4, et
- R et R', identiques ou différents, représentent chacun un atome d'hydrogène ou un radical alkyle ayant de 1 à 5 atomes de carbone en chaîne droite ou ramifiée, ou
- R et R' forment ensemble avec l'atome d'azote auquel ils sont liés un radical hétérocyclique penta ou hexagonal renfermant éventuellement un atome d'oxygène ou un deuxième atome d'azote lequel peut être lui-même substitué par un radical alkyle renfermant de 1 à 5 atomes de carbone en chaîne droite ou ramifiée ou par un radical arylalkyle dans lequel le groupe alkyle renferme de 1 à 5 atomes de carbone et le groupe aryle est éventuellement mono- ou poly-substitué par un atome d'halogène ou un radical alkyle ou alkoxy ayant chacun de 1 à 5 atomes de carbone et

* un des R₁, R₂, et R₃ représente un atome d'hydrogène ou un radical méthyle.
   Ces composés et leurs sels physiologiquement tolérables sont utilisables comme médicaments dans le traitement des pathologies caractérisés par une perte du tissu osseux.

## Description

La présente invention a pour objet de nouveaux composés du thiophène, leur procédé de préparation et les compositions pharmaceutiques qui les contiennent.

Elle concerne plus particulièrement les composés du thiophène de formule I: 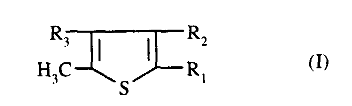 dans laquelle :
* un des R₁, R₂ et R₃ représente le radical de formule 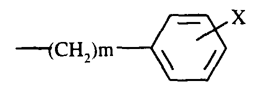 dans laquelle :
   m représente un nombre entier de 2 à 6 inclus et
   X représente un atome d'hydrogène, un radical alkyle ou alkoxy ayant chacun de 1 à 5 atomes de carbone en chaîne droite ou ramifiée, ou un radical dialkylamino dans lequel chaque groupe alkyle renferme de 1 à 5 atomes de carbone,
* un des R₁, R₂ et R₃ représente le radical de formule 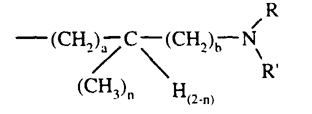 dans laquelle :
   - n représente 0, 1 ou 2 ;
   - a représente 2 ou 3 et
   - b représente 1 ou 2 de telle sorte que a + b = 4, et
   - R et R', identiques ou différents, représentent chacun un atome d'hydrogène ou un radical alkyle ayant de 1 à 5 atomes de carbone en chaîne droite ou ramifiée, ou
   - R et R' forment ensemble avec l'atome d'azote auquel ils sont liés un radical hétérocyclique penta ou hexagonal renfermant éventuellement un atome d'oxygène ou un deuxième atome d'azote lequel peut être lui-même substitué par un radical alkyle renfermant de 1 à 5 atomes de carbone en chaîne droite ou ramifiée ou par un radical arylalkyle dans lequel le groupe alkyle renferme de 1 à 5 atomes de carbone et le groupe aryle est éventuellement mono- ou poly-substitué par un atome d'halogène ou un radical alkyle ou alkoxy ayant chacun de 1 à 5 atomes de carbone et
* un des R₁, R₂ et R₃ représente un atome d'hydrogène ou un radical méthyle

L'état antérieur de la technique le plus proche de la présente invention est illustré notamment par le brevet EPO 429 370 qui concerne des composés du thiophène de formule : 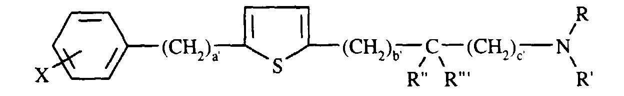 qui ont une activité anti-résorbante osseuse, mais ne suggèrent nullement les composés de la présente invention.

La présente invention a également pour objet le procédé de préparation des composés de formule I caractérisé en ce que :
* l'on transforme l'acide de formule II : 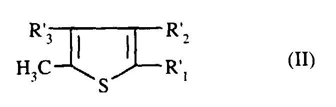 dans laquelle :
   - un des R'₁, R'₂ et R'₃ représente le radical de formule 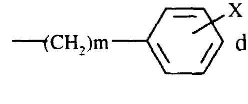 dans laquelle m et X ont les significations précédemment définies ;
   - un des R'₁, R'₂ et R'₃ représente le radical de formule 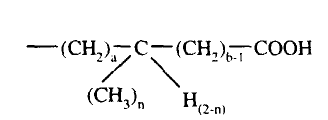 dans laquelle n, a et b ont les significations précédemment définies, et
   - un des R'₁, R'₂ et R'₃ représente un atome d'hydrogène ou un radical méthyle,
   en l'amide correspondante de formule III : 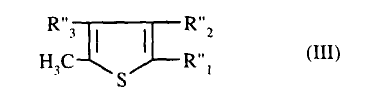 dans laquelle :
   - un R"₁, R"₂, et R"₃ représente le radical de formule 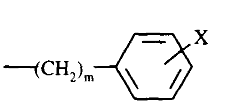 dans laquelle m et X ont les significations précédemment définies ,
   - un des R"₁, R"₂ et R"₃ représente le radical de formule 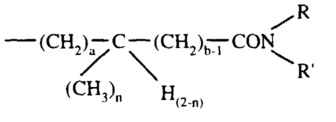 dans laquelle n, a, b, R et R' ont les significations précédemment définies, et
   - un des R"₁, R"₂ et R"₃ représente un atome d'hydrogène ou un radical méthyle ;
* et l'on réduit l'amide de formule III pour obtenir les composés correspondants de formule I.

La transformation de l'acide de formule II en amide de formule III s'effectue selon les méthodes classiques connues de la littérature telles que par exemple la transformation de l'acide en chlorure d'acide correspondant lequel sert à acvler l'amine de formule : 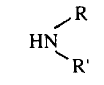 dans laquelle R et R' ont les significations précédemment définies.

L'acylation s'effectue en présence d'un accepteur de l'acide chlorhydrique formé au cours de la réaction ; cet accepteur pouvant être, par exemple, un excès de l'amine utilisée pour la réaction.

La réduction de l'amide de formule III s'effectue de façon particulièrement convenable au moyen de LiAlH₄ en opérant à reflux dans un solvant adéquat tel que par exemple l'éther.

Les acides de départ de formule II ont été préparés à partir de matières premières connues selon des procédés connus comme décrit ci-après.

Les composés de formule I donnent des sels avec des acides physiologiquement tolérables - sels qui sont, à ce titre, inclus dans la présente invention.

Les composés de la présente invention possèdent des propriétés pharmacologiques et thérapeutiques intéressantes notamment sur le métabolisme osseux, tout en étant dépourvus de toxicité, ce qui permet de les utiliser dans les pathologies caractérisées par une perte du tissu osseux, comme l'ostéoporose, la maladie de Paget, la parodontite et la polyarthrite rhumatoïde.

La présente invention a également pour objet les compositions pharmaceutiques contenant comme principe actif un composé de formule générale I ou un de ses sels physiologiquement tolérable, mélangé ou associé à un excipient pharmaceutique approprié, comme par exemple, le glucose, le lactose, l'amidon, le talc, l'éthyl cellulose, le stéarate de magnésium ou le beurre de cacao.

Les compositions pharmaceutiques ainsi obtenues se présentent généralement sous forme dosée et peuvent contenir de 0,1 à 10 mg de principe actif.

Elles peuvent revêtir la forme de comprimés, dragées, gélules, suppositoires, solutions injectables ou buvables et être, selon les cas, administrées par la voie orale, rectale ou parentérale.

La posologie varie notablement selon l'age et le poids du patient, la voie d'administration, la nature de la maladie et les traitements associés et s'échelonnent de 0,2 à 20 mg de principe actif, 1 à 2 fois par jour.

Les exemples suivants illustrent la présente invention. Les points de fusion sont déterminés, sauf mention contraire, à la platine chauffante de Kofler.

### I/ Synthèse des matières premières de formule II

### A) 2-méthyl-3-(4-carboxy-3,3-diméthylbutyl)-5-(3-p-méthylphényl propyl)thiophène, (A1) et 2-méthyl-4-(4-carboxy-3,3-diméthylbutyl)-5-(3-p-méthylphényl propyl) thiophène, (A2) :

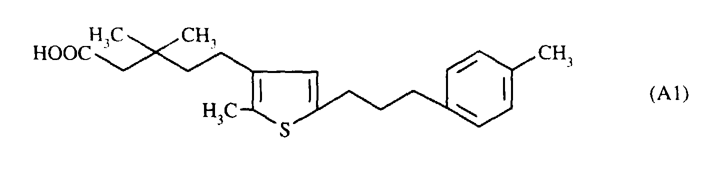

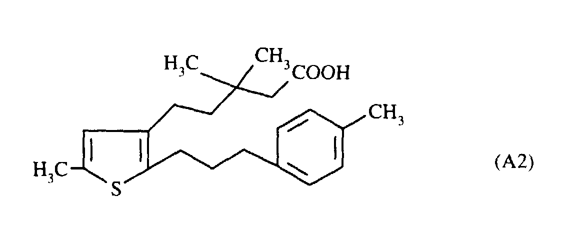

### Stade 1 : 2-méthyl-5-(p-méthylphényl propionyl)thiophène

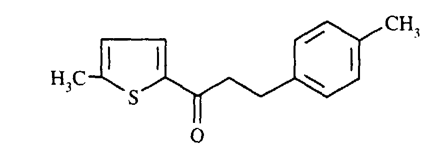

Dans un ballon tricol de 1 litre muni d'une agitation mécanique et d'un réfrigérant, on place 160,65 g (1,35 mole) de chlorure de thionyle. On y ajoute peu à peu 164,2 g (0,9 mole) d'acide p-méthylphényl propionique, à température ambiante. Après 4 heures, la réaction est amenée à son terme par chauffage à 60 °C, pendant 1 heure. L'excès de SOCl₂ est éliminé par distillation sous vide. On obtient ainsi 164,5 g de chlorure de l'acide p-méthylphényl propionique.

Dans un ballon tricol de 3 litre, muni d'une garde à CaCl₂, d'une agitation mécanique, d'un thermomètre et d'une ampoule à brome, on place 91 g (0,99 mole) de 2-méthylthiophène, 1,4 l de dichlorométhane et le chlorure d'acide préalablement préparé.

Le mélange réactionnel est amené à 5 °C sur bain de glace puis additionné de 281,3 g (1,08 mole) de SnCl₄ en 40 minutes en maintenant l'ensemble à une température inférieure à 5 °C. Puis après 12 heures à température ambiante, le milieu est hydrolysé sur un mélange eau/glace/HCl concentré (1 litre/500 g/170 ml). La phase organique est décantée et la phase aqueuse extraite à CH₂Cl₂.

Les phases organiques réunies sont lavées par HCl dilué puis H₂O, traitées par MgSO₄ et noir decarbone puis concentrées. Le résidu, recristallisé dans le cyclohexane, donne 173 g du produit attendu sous forme d'un solide blanc fondant à 52 °C. Rendement : 79 %.

### Stade 2: 2-méthyl-5-(p-méthylphényl propyl)thiophène

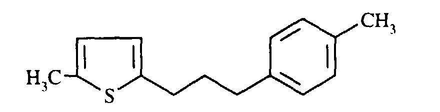

Dans un ballon tricol de 3 litres muni d'une agitation mécanique, d'un réfrigérant et d'un thermomètre, on introduit 90 g (0,369 mole) de 2-méthyl-5-(p-méthylphényl propionyl)thiophène et 965 ml de triéthylèneglycol.

Le mélange réactionnel est tiédi à 55 °C et 64,30 g (1,27 mole) d'hydrate d'hydrazine y sont ajoutés en une fois. La température est amenée à 90 °C et 63,2 g (1,127 mole) de potasse en pastille sont ajoutés au mélange. Le mélange réactionnel est porté à un fort reflux et l'eau est distillée. La température croit jusqu'à 220 °C et est maintenue ainsi pendant 45 minutes.

Après refroidissement à 50 °C, on ajoute 900 ml d'eau et 155 ml d'HCl concentré. Le mélange est extrait à l'éther. Les phases organiques sont lavées successivement par HCl dilué, H₂O, NaOH dilué et H₂O puis séchées sur MgSO₄, décolorées au noir de carbone et concentrées.

L'huile obtenue est distillée sous vide (13,33 Pa). On obtient 77,5 g du produit attendu sous forme d'une huile incolore P Eb/_{13,33Pa} = 110-115 °C. Rendement : 91 %.

### Stade 3 : 2-méthyl-3-(4-éthoxycarbonyl-3,3-diméthyl butanoyl)-5-(p-méthylphényl propyl)thiophène (A'1) et 2-méthyl-4-(4-éthoxycarbonyl-3,3-diméthyl butanoyl)-5-(p-méthylphényl propyl)thiophène (A'2)

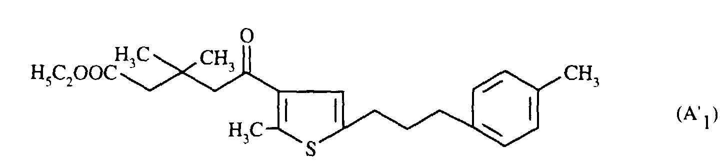

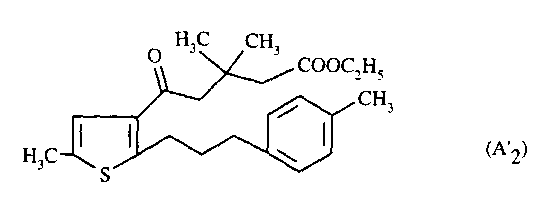

Dans un ballon tricol de 250 ml muni d'une agitation mécanique et d'un thermomètre, on place 15 g (0,0651 mole) de 2-méthyl-5-(p-méthylphényl propyl)thiophène, 10,2 g (0,0716 mole) d'anhydride 3,3-diméthyl glutarique et 115 ml de nitrobenzène. Le mélange est amené à 0 °C sur bain de glace et 13 g (0,0976 mole) de chlorure d'aluminium sont ajoutés par fractions.

Après 1 heure à 0 °C et 2 heures à température ambiante, le mélange est hydrolysé sur un mélange eau/glace/HCl concentré (90 g/90 g/18 ml) puis décanté. Le nitrobenzène est éliminé par entraînement à la vapeur et le résidu est extrait par CH₂Cl₂. La phase organique est lavée par HCl dilué puis H2O, séchée sur MgSO4, décolorée au noir de carbone et concentrée. L'huile obtenue est constituée d'un mélange constitué pour 51 % de 2-méthyl-3-(4-carboxy-3,3-diméthyl butanoyl)-5-(p-méthylphényl propyl)thiophène et pour 49 % de 2-méthyl-4-(4-carboxy-3,3-diméthyl butanoyl)-5-(p-méthylphényl propyl)thiophène.

Le mélange d'acides bruts est estérifié par action de l'éthanol en présence d'acide p-toluènesulfonique. Après traitement usuels, le mélange d'esters est soumis à une chromatographie sur silice (éluant : CH₂Cl₂). Les fractions convenables réunies et concentrées laissent :
. 10,17 g d'une huile incolore identifiée à l'ester de formule A'₁ (Rendement : 39 %) ;
. 8 g d'une huile incolore constituée d'un mélange des esters A'₁ et A'₂ ; et
. 3,9 g d'une huile incolore identifiée à l'ester de formule A'₂ (Rendement : 15 %).

### Stade 4 : Produits titre (A₁ et (A₂)

Dans un ballon tricol de 250 ml muni d'une agitation mécanique, d'un réfrigérant et d'un thermomètre, on place 7,7 g (0,0192 mole) de 2-méthyl-3-(4-éthoxycarbonyl-3,3-diméthylbutanoyl)-5-(p-méthylphényl propyl)thiophène (A'₁) et 80 ml de triéthylèneglycol.

Le mélange réactionnel est amené à 60 °C et 3,35 g (0,067 mole) d'hydrate d'hydrazine y sont ajoutés en une fois. La réaction est portée à un fort reflux. L'eau est distillée et la température est amenée à 210 °C pendant 35 minutes. Après refroidissement, le milieu est hydrolysé par 120 ml d'eau et 25 ml d'HCl concentré, puis décanté. La phase aqueuse est extraite par CH₂Cl₂. Les phases organiques sont lavées par HCI dilué puis H₂O, séchée sur MgSO₄, décolorée au noir de carbone et concentrée.

On obtient 9,3 g de 2-méthyl-3-(4-carboxy-3,3-diméthylbutyl)-5-(3-p-méthylphényl propyl)thiophène, sous la forme d'une huile jaune pâle, Rendement : 91 %.

En opérant de la même façon, mais en employant l'ester de formule A'2 à la place de l'ester de formule A'1, a été obtenu le 2-méthyl-4-(4-carboxy-3,3-diméthyl butyl)-5-(3-p-méthylphényl propyl)thiophène.

### B) 2,3-diméthyl-4-(3-p-méthylphényl propyl)-5-(4-carboxy-3,3-diméthylbutyl) thiophène

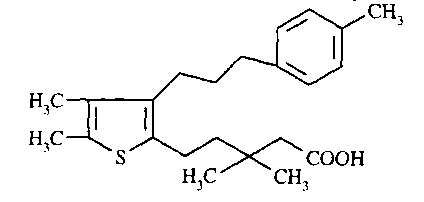

### Stade 1: 2,3-diméthyl-5-(4-carboxy-3,3-diméthyl butanoyl)thiophène

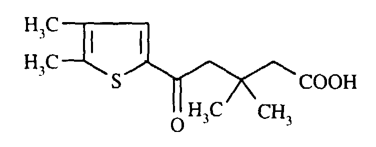

Dans un ballon tricol de 2 litres muni d'une agitation mécanique, on introduit 40 g (0,355 mole) de 2,3-diméthyl thiophène, 630 ml de nitrobenzène et 55,5 g (0,390 mole) d'anhydride 2,3-diméthylglutarique. Le milieu est amené à 0 °C et 71 g (0,532 mole) de chlorure d'aluminium sont ajoutés par fractions en maintenant la température sous 5 °C. Après 10 heures d'agitation à température ambiante, le mélange est hydrolysé sur eau/glace/HCl concentré (500 g/500 g/170 ml) pendant 15 minutes. Puis le nitrobenzène est entraîné à la vapeur, et le résidu extrait par 3 fois 100 ml de CH₂Cl₂. Les phases organiques réunies sont lavées par HCl dilué puis H₂O, séchées sur MgSO₄, décolorées au noir de carbone et concentrées pour donner 73,4 g de 2,3-diméthyl-5-(4-carboxy-3,3-diméthyl butanoyl)thiophène sous forme d'une huile jaune pâle, Rendement : 81 %.

### Stade 2: 2,3-diméthyl-5-(4-carboxy-3,3-diméthyl)butyl)thiophène

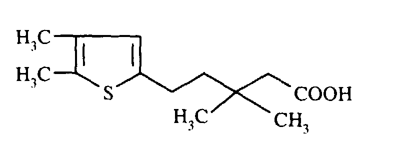

Dans un ballon tricol de 3 litres muni d'une agitation, d'un réfrigérant et d'un thermomètre, on introduit 72,9 g (0,29 mole) de 2,3-diméthyl-5-(4-carboxy-3,3-diméthyl butanoyl)thiophène et 580 ml de triéthylène glycol. Après avoir tiédi le mélange vers 40 °C, on y ajoute en une fois 50,03 g (1 mole) d'hydrazine monohydratée. Le mélange réactionnel est alors chauffé jusqu'à 90 °C et 49,2 g (0,88 mole) de potasse en pastilles y sont ajoutés. L'ensemble est chauffé jusqu'à un fort reflux en 30 minutes (145 °C) ; l'eau est distillée ; la température croît jusqu'à 200 °C et y est maintenue pendant 1 heure.

Après refroisissement à 50 °C, on ajoute 1,4l d'eau puis 100 ml d'HCl concentré. La phase aqueuse est extraite à l'éther ; la phase éthérée est lavée par HCl dilué, H₂O puis traitée par MgSO₄ et au noir de carbone. Après concentration, le résidu est cristallisé dans l'éther de pétrole, filtré, essoré et séché. On obtient 49 g de 2,3-diméthyl-5-(4-carboxy-3,3-diméthylbutyl thiophène, sous forme d'un solide blanc, Rendement : 60 %.

### Stade 3 : 2,3-diméthyl-5-(4-éthoxycarbonyl-3,3-diméthyl butyl)thiophène

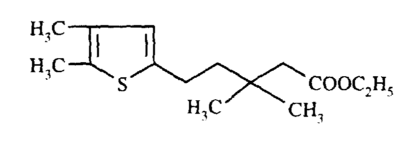

Dans un ballon tricol de 500 ml muni d'une agitation mécanique, d'un réfrigérant et d'un thermomètre, on introduit 30 g (0,125 mole) de 2,3-diméthyl 5-(4-carboxy 3,3-diméthyl)butyl thiophène, 60 ml de diméthylformamide et 20,73 g (0,15 mole) de carbone de potassium.

Après avoir tiédi le mélange vers 40 °C, on y ajoute, en 30 minutes, 21,45 g (0,137 mole) d'iodure de méthyle. Le mélange réactionnel est maintenu à 80 °C pendant 10 heures puis concentré sous vide et repris par 250 ml d'éther anhydre. L'iodure de potassium est éliminé par filtration. La phase éthérée est lavée à l'eau, séchée sur MgSO4, décolorée au noir de carbone et concentrée. On obtient 32,96 g de 2,3-diméthyl-5-(4-éthoxycarbonyl-3,3-diméthyl butyl)thiophène sous forme d'une huile jaune, Rendement : 98 %.

### Stade 4 : 2,3-diméthyl-4-(3-p-méthylphényl propionyl)-5-(4-éthoxycarbonyl-3,3-diméthyl butyl)thiophène

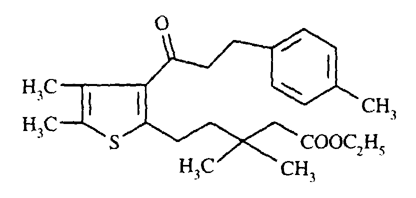

Dans un ballon tricol de 250 ml, on introduit 15,3 g (0,056 mole) de 2,3-diméthyl-5-(4-éthoxycarbonyl-3,3-diméthyl butyl)thiophène, le chlorure d'acide préparé à partir de9,2 g (0,056 mole) d'acide p-méthylphényl propionique et de 6,1 ml (0,084 mole) de SOCl₂, et 90 ml de CH₂Cl₂.

Le mélange réactionnel est amené à 0 °C puis additionné, goutte à goutte en 25 minutes, de 8 ml (0,0672 mole) de SnCl₄ tout en maintenant la température en dessous de 5 °C.

Après 10 heures d'agitation, le mélange réactionnel est hydrolysé sur eau/glace/HCl concentré (90 g/ 90 g/11 ml) et extrait par 3 fois 100 ml de CH₂Cl₂.

Les phases organiques sont lavées à l'eau et au bicarbonate de sodium, séchées sur MgSO₄ et décolorées au noir de carbone. Après distillation du solvant, on recupère 21,74 g du produit attendu sous forme d'une huile jaune pâle (Rendement 94 %) dont un échantillon est purifié par chromatographie sur silice (éluant : cyclohexane/CH₂Cl₂, 80/20) pour donner un échantillon analytique pur.

### Stade 5 : Produit titre

Dans un ballon tricol de 500 ml, on met en solution dans 50 ml de triéthylèneglycol, 10,5 g (0,025 mole) du produit obtenu au stade 4. On porte la solution à 80 °C puis y ajoute 4,36 g (0,0863 mole) d'hydrate d'hydrazine. La température est alors maintenue à 95 °C puis on ajoute au mélange 4,29 g (0,0765 mole) de potasse.

Après 15 minutes de reflux (≈ 130 °C), l'eau formée est distillée. Lorsque la température atteint 230 °C, le milieu réactionnel est refroidi à 20 °C, hydrolysé par 125 ml d'eau, acidifié par 90 ml d'HCl concentré et extrait par 4 fois 25 ml d'éther éthylique. Les phases organiques sont lavées par HCI dilué puis H₂O, séchées sur MgSO₄ et décolorées au noir de carbone. Le résidu concentré est chromatographié sur silice (éluant : CH₂Cl₂/CH₃COOC₂H₅, 98/2). Après concentration, on obtient 6,2 g de 2,3-diméthyl-4-(3-p-méthylphényl propyl)-5-(4-carboxy-3,3-diméthyl butyl)thiophène, sous forme d'une huile incolore, Rendement : 70 %

### C) 2-méthyl-3-(3-p-méthylphényl propyl)-5-(4-carboxy-3,3-diméthyl butyl)thiophène et 2-méthyl-4-(3-p-méthylphényl propyl)-5-(4-carboxy-3,3-diméthyl butyl)thiophène.

Ces deux composés ont été préparés selon la méthode décrite au paragraphe B), à partir du 2-méthylthiophène et après séparation des esters intermédiaires de formule A"₁ et A"₂.

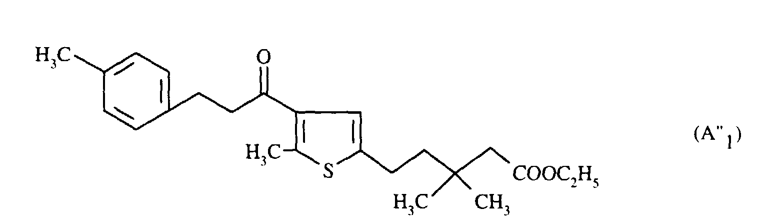

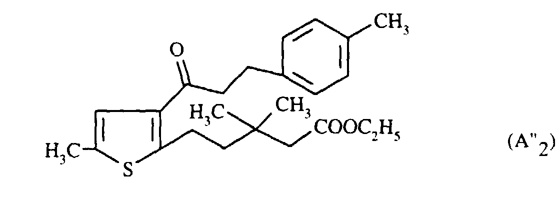

### D) 2,5-diméthyl-3-(3-phényl propyl)-4-(4-carboxy-3,3-diméthyl butyl)thiophène

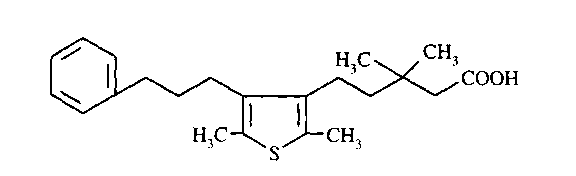

### Stade 1: 2,5-diméthyl-4-(4-méthoxycarbonyl-3,3-diméthyl butyl)thiophène

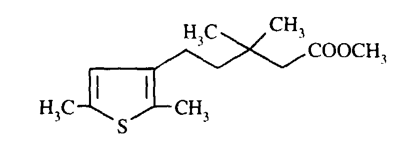

Ce produit a été préparé par analogie à la préparation du produit objet du paragraphe B) stade 3, en utilisant comme matière première le 2,5-diméthyl thiophène.

### Stade 2 : 2,5-diméthvl-3-(3-oxo-3-phényl propyl)-4-(4-méthoxycarbonyl 3,3-diméthyl butyl)thiophène

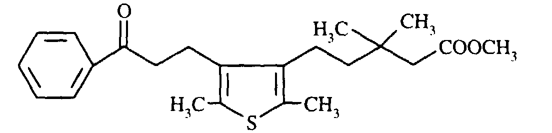

Dans un ballon tricol de 250 ml muni d'un agitateur magnétique, d'un thermomètre et d'une ampoule à brome, on place 8,36 g (0,0328 mole) de 2,5-diméthyl-4-(4-méthoxycarbonyl-3,3-diméthyl butyl)thiophène, 7 g (0,0328 mole) de 3-bromo propionyl benzène et 120 ml de dichlorométhane. En maintenant une température inférieure à 5 °C, on coule 10,7 g (0,0410 mole) de SnCl₄. Après 12 heures à température ambiante, on hydrolyse sur un mélange eau/glace/HCl concentré (150 g/150 g/50 ml). La phase organique est lavée par HCl dilué, H₂O, puis séchée sur MgSO₄, décolorée au noir de carbone et concentrée. Le résidu est chromatographié sur silice (éluant : CH₂Cl₂). Les fractions adéquates, concentrées donnent 5,85 g de l'ester attendu sous forme d'une huile incolore, Rendement : 46 %.

### Stade 3 : Produit titre

Dans un ballon tricol de 250 ml, muni d'une agitation mécanique, d'un réfrigérant et d'un thermomètre, on place 6,4 g (0,0166 mole) de 2,5-diméthyl-3-(3-oxo-3-phényl propyl)-4-(4-méthoxycarbonyl-3,3-diméthyl butyl)thiophène et 100 ml de triéthylèneglycol. La température est amenée à 60 °C et on ajoute au mélange réactionnel 1,86 g (0,0580 mole) d'hydrate d'hydrazine à 80 %, puis à 90 °C on ajoute 2,84 g (0,050 mole) de potasse. Le mélange réactionnel est alors porté à un fort reflux, et après distillation de l'eau, la température est maintenue à 210 °C pendant 45 minutes.

Après refroidissement, le mélange est hydrolysé sur un mélange eau/glace/HCl concentré (200 g/100 g/17 ml) puis extrait à l'éther. Les phases organiques réunies sont lavées par HCl dilué, H₂O puis séchées sur MgSO₄, décolorées ou noir de carbone, et concentrées pour donner 4,5 g de 2,5-diméthyl-3-(3-phényl propyl)-4-(4-carboxy-3,3-diméthyl butyl)thiophène, sous forme d'une huile jaune pâle, Rendement : 75 %.

### II/ Synthèse des composés de formule I

### Exemple 1:

2,3-diméthyl-4-(3-p-méthylphényl propyl)-5-(5-morpholino-3,3-diméthylpentyl) thiophène 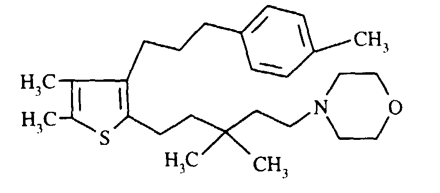 et son chlorhydrate.

### Stade 1 : 2,3-diméthyl-4-(3-p-méthylphényl propyl)-5-(4-morpholinocarbonyl-3,3-diméthyl butyl)thiophène

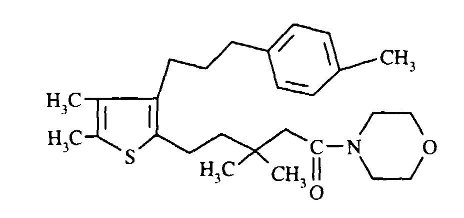

Dans un ballon monocol de 100 ml, on place 2 g (0,0157 mole) de chlorure d'oxyallyle en solution dans 10 ml de CHCl₃. Après avoir amené le milieu à 0 °C sur bain de glace, on additionne 3,9 g (0,0105 mole) de 2,3-diméthyl-4-(3-p-méthylphényl propyl)-5-(4-carboxy-3,3-diméthyl butyl)thiophène (cf ci-dessus le paragraphe B de la synthèse des matières premières).

Le mélange réactionnel est alors agité à température ambiante puis à 50 °C jusqu'à la fin du dégagement gazeux. Le solvant est distillé à température inférieure à 50 °C.

Dans un ballon tricol de 500 ml, on coule simultanément sous agitation le chlorure d'acide ci-dessus préparé en solution dans 60 ml d'éther et 1,82 g (0,021 mole) de morpholine en solution dans 60 ml d'éther. Le chlorhydrate de morpolinium précipite instantanément.

Après une heure d'agitation, le solide est éliminé par filtration. Le filtrat est lavé à l'eau, séché sur MgSO₄, décoloré au noir de carbone et concentré à sec.

Le produit brut obtenu est purifié par chromatographie sur silice (éluant : CH₂Cl₂/CH₃COOC₂H₅ - 95/5, puis CH₂Cl₂/CH₃OH - 98/2). On obtient ainsi 3,31 g de2,3-diméthyl-4-(3-p-méthylphényl propyl)-5-(4-morpholinocarbonyl-3,3-diméthyl butyl) thiophène, sous forme d'une huile incolore, Rendement : 72 %.

### Stade 2 : Produit titre

Dans un ballon tricol de 50 ml équipé d'une agitation magnétique, d'un réfrigérant et d'un balayage d'azote, on met en suspension 0,45 g (0,0119 mole) de LiAlH₄ dans 12 ml d'éther éthylique. On additionne 2,56 g (0,0058 mole) de l'amide préparée au stade 1 ci-dessus en solution dans 12 ml d'éther en 30 minutes environ. La réaction est exothermique. Le milieu réactionnel est maintenu à reflux pendant une heure, puis refroidi à 20 °C et hydrolysé par H₂O (0,45 ml), NaOH 4N (0,45 ml) et H₂O (0,9 ml). Le solide est éliminé par filtration et lavé à l'éther. Les phases organiques sont lavées à l'eau, séchées sur MgSO₄ et décolorées sur noir de carbone.

Le chlorhydrate du produit titre est précipité par ajout d'un équivalent d'HCl en solution dans l'éther, essoré et séché sous vide. On obtient 1,64 g de chlorhydrate de 2,3-diméthyl-4-(3-p-méthylphényl propyl)-5-(5-morpholino-3,3-diméthylpentyl)thiophène, sous forme d'un solide de blanc cristallisé, PF : 138 °C, Rendement : 61 %.

### Exemples 2-9 :

En opérant selon le mode opératoire décrit dans l'exemple 1 à partir des acides adéquats dont la préparation a été décrite au paragraphe I, ont été préparés les composés objet des exemples suivants :
2) 2,5-diméthyl-3-(3-phényl propyl)-4-(5-morpholino-3,3-diméthyl pentyl)thiophène et son chlorhydrate fondant à 167 °C.
3) 2,5-diméthyl-3-(3-phényl propyl)-4-{5-[4-(2,3,4-triméthoxybenzyl)pipérazin-1-yl] 3,3-diméthyl pentyl}thiophène, et son dichlorhydrate fondant à 165 °C (pureté : 98 %).
4) 2,3-diméthyl-4-(3-p-méthylphényl propyl)-5-{5-[4-(2,3,4-triméthoxybenzyl)pipérazin-1-yl]-3,3-diméthyl pentyl}thiophène, et son dichlorhydrate fondant à 150 °C (pureté : 99 %).
5) 2-méthyl-3-{5-[4-(2,3,4-triméthoxy benzyl)pipérazin-1-yl]-3,3-diméthyl pentyl} 5-(3-p-méthylphényl propyl)thiophène, et son dichlorhydrate fondant à 200 °C (pureté : 96 %).
6) 2-méthyl-4-(5-morpholino-3,3-diméthyl pentyl)-5-(3-p-méthylphényl propyl) thiophène, et son chlorhydrate fondant à 164 °C (pureté : 98 %).
7) 2-méthyl-4-{5-[4-(2,3,4-triméthoxy benzyl)pipérazin-1-yl]-3,3-diméthyl pentyl}-5-(3-p-méthylphényl propyl}thiophène et son dichlorhydrate fondant à 180 °C (pureté : 99 %).
8) 2-méthyl-3-(3-p-méthylphényl propyl)-5-{5-[4-(2,3,4-triméthoxy benzyl)pipérazin-1-yl]-3,3-diméthyl pentyl}thiophène, et son dichlorhydrate fondant à 200 °C (pureté 99 %).
9) 2-méthyl-4-(3-p-méthylphényl propyl)-5-{5-[4-(2,3,4-triméthoxybenzyl)pipérazin-1-yl]-3,3-diméthyl pentyl}thiophène, et son dichlorhydrate fondant à 170 °C (pureté : 98 %).

### Exemple 10

### Etude pharmacologique

Les composés de la présente invention possèdent des propriétés pharmacologiques intéressantes notamment sur le métabolisme osseux.

A l'état normal, le remodelage osseux permet d'assurer l'intégrité anatomique et structurale du squelette. Il s'opère par cycles et dépend de deux grands types de population cellulaire, les ostéoclastes et les ostéoblastes. Les ostéoclastes, après activation, assurent la résorption de l'os ancien par acidification puis digestion enzymatique, laissant place, dans une phase successive, aux ostéoblastes pour former le nouveau tissu ostéoïde qui sera ensuite calcifié.

Ces deux phases métaboliques -résorption et formation- sont à l'état physiologique étroitement couplées l'une à l'autre. En situation pathologique, un déséquilibre de ces deux phases peut se produire, avec en particulier une hyperactivité de résorption entraînant une perte osseuse excessive, insuffisamment compensée par la phase de néoformation. C'est en particulier le cas de l'ostéoporose post-ménopausique.

L'activité des composés décrits a été mise en évidence en particulier sur un test pratiqué sur tissu isolé de calottes crâniennes de souris - dont la résorption osseuse est stimulée par l'acide rétinoïque. La méthode utilisée est directement inspirée de la technique décrite par J.J. REYNOLDS et coll. Calc. Tiss. Tes. 4, 339-49 (1970).

Dans cette situation d'hyperrésorption, les composés utilisés à des concentrations molaires comprises entre 10⁻⁶ et 10⁻⁵ ont entrainé des variations allant de -3 à -45 %. A titre d'illustration, les composés des exemples n° 2 et 3, particulièrement représentatifs de l'invention, ont donné les résultats suivants :

| **Composés** | **Concentration molaire** | **% variation** |
|---|---|---|
| *Exemple* 2 | 10⁻⁶ | -3 % |
| | 5.10⁻⁶ | -13% |
| | 10⁻⁵ | -17 % |
| *Exemple 3* | 10⁻⁶ | -7 % |
| | 5.10⁻⁶ | -31 % |
| | 10⁻⁵ | -42 % |

Ces composés présentent ainsi des propriétés antirésorbantes intéressantes permettant leur utilisation thérapeutique dans les pathologies caractérisées par une perte ossseuse en particulier lorsque celle-ci est due à un excès de résorption.

## Revendications

1. Les composés du thiophène de formule I: 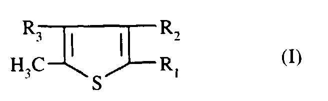 dans laquelle :
* un des R₁, R₂ et R₃ représente le radical de formule 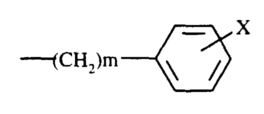 dans laquelle :
m représente un nombre entier de 2 à 6 inclus et
X représente un atome d'hydrogène, un radical alkyle ou alkoxy ayant chacun de 1 à 5 atomes de carbone en chaîne droite ou ramifiée, ou un radical dialkylamino dans lequel chaque groupe alkyle renferme de 1 à 5 atomes de carbone,
* un des R₁, R₂ et R₃ représente le radical de formule 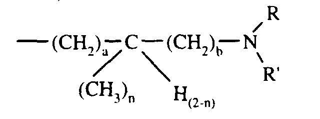 dans laquelle :
- n représente 0, 1 ou 2 ;
- a représente 2 ou 3 et
- b représente 1 ou 2 de telle sorte que a + b = 4, et
- R et R', identiques ou différents, représentent chacun un atome d'hydrogène ou un radical alkyle ayant de 1 à 5 atomes de carbone en chaîne droite ou ramifiée, ou
- R et R' forment ensemble avec l'atome d'azote auquel ils sont liés un radical hétérocyclique penta ou hexagonal renfermant éventuellement un atome d'oxygène ou un deuxième atome d'azote lequel peut être lui-même substitué par un radical alkyle renfermant de 1 à 5 atomes de carbone en chaîne droite ou ramifiée ou par un radical arylalkyle dans lequel le groupe alkyle renferme de 1 à 5 atomes de carbone et le groupe aryle est éventuellement mono- ou poly-substitué par un atome d'halogène ou un radical alkyle ou alkoxy ayant chacun de 1 à 5 atomes de carbone et
* un des R₁, R₂ et R₃ représente un atome d'hydrogène ou un radical méthyle et leurs sels physiologiquement tolérables avec des acides appropriés.

2. Un composé de la revendication 1 qui les le 2,3-diméthyl-4-(3-p-méthylphényl propyl)-5-(5-morpholino-3,3-diméthyl pentyl)thiophène, et son chlorhydrate.

3. Le procédé de préparation des composés de la revendication 1 caractérisé en ce que :
* l'on transforme l'acide de formule II : 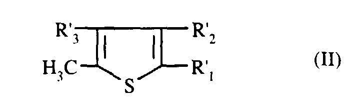 dans laquelle :
- un des R'₁, R'₂ et R'₃ représente le radical de formule 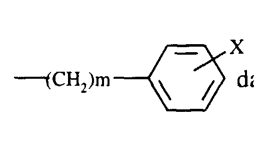 dans laquelle m et X ont les significations définies dans la revendication 1;
- un des R'₁, R'₂ et R'₃ représente le radical de formule 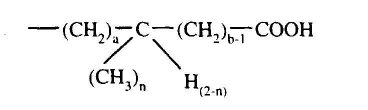 dans laquelle n, a et b ont les significations définies dans la revendication 1, et
- un des R'₁, R'₂ et R'₃ représente un atome d'hydrogène ou un radical méthyle,
en l'amide correspondante de formule III : 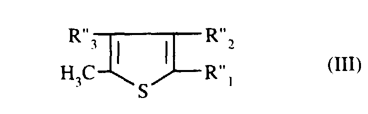 dans laquelle :
- un R" ₁, R"₂, et R"₃ représente le radical de formule 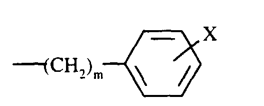 dans laquelle m et X ont les significations définies dans la revendication 1,
- un des R"₁, R"₂ et R"₃ représente le radical de formule 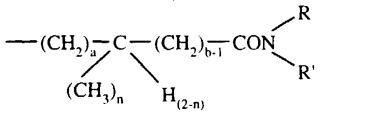 dans laquelle n, a, b, R et R' ont les significations définies dans la revendication 1, et
- un des R"₁, R"₂ et R"₃ représente un atome d'hydrogène ou un radical méthyle ;
* et l'on réduit l'amide de formule III pour obtenir les composés correspondants de formule I.

4. Les compositions pharmaceutiques agissant sur le métabolisme osseux contenant comme principe actif un composé de la revendication 1 mélangé aou associé à un excipient pharmaceutique approprié.

5. L'utilisation des compositions de la revendication 4 dans le traitement des pathologies caractérisées par une perte du tissu osseux.
